(19) Europäisches Patentamt
European Patent Office
Office européen des brevets

(11) EP 2 320 227 B1

(12) **EUROPEAN PATENT SPECIFICATION**

(45) Date of publication and mention
of the grant of the patent:
**20.02.2013  Bulletin 2013/08**

(51) Int Cl.:
*G01N 33/15* (2006.01)       *G01N 13/00* (2006.01)
*B01F 1/00* (2006.01)        *B01F 11/00* (2006.01)

(21) Application number: **09175011.7**

(22) Date of filing: **04.11.2009**

(54) **Dissolution apparatus comprising beads and process**

Auflösungsvorrichtung mit Kügelchen und Verfahren

Appareil de dissolution comportant des billes et processus

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(43) Date of publication of application:
**11.05.2011  Bulletin 2011/19**

(73) Proprietor: **LEK Pharmaceuticals d.d.**
**1526 Ljubljana (SI)**

(72) Inventors:
  • **Klancar, Uros**
    **1526 Ljubljana (SI)**
  • **Legen, Igor**
    **1526 Ljubljana (SI)**

(74) Representative: **Prüfer & Partner GbR**
**European Patent Attorneys**
**Sohnckestrasse 12**
**81479 München (DE)**

(56) References cited:
**GB-A- 2 136 123**

• **AOKI S ET AL: "Determination of the mechanical
impact force in the in vitro dissolution test and
evaluation of the correlation between in vivo and
in vitro release" INTERNATIONAL JOURNAL OF
PHARMACEUTICS, ELSEVIER BV, NL, vol. 95, no.
1-3, 30 June 1993 (1993-06-30), pages 67-75,
XP025568686 ISSN: 0378-5173 [retrieved on
1993-06-30]**
• **MACHIDA Y ET AL: "A NEW METHOD OF
DISSOLUTION TESTING FOR OILY DRUG
PREPARATIONS USING AN IMPROVED
APPARATUS" CHEMICAL AND
PHARMACEUTICAL BULLETIN,
PHARMACEUTICAL SOCIETY OF JAPAN,
TOKYO, JP, vol. 34, no. 6, 1 June 1986
(1986-06-01), pages 2637-2641, XP001181375
ISSN: 0009-2363**

**Description**

Field of the invention

The present invention relates to an apparatus for testing dissolution of an active pharmaceutical ingredient. Furthermore, the present invention relates to a process for testing dissolution of an active pharmaceutical ingredient and a process for assaying or predicting in vivo dissolution of an active pharmaceutical ingredient.

Description of background art

Generally, dissolution testing involves measuring the rate of an active pharmaceutical ingredient (API) release from the dosage form by determining the amount of API that goes into testing media as a function of time. It is commonly employed using a pharmacopeial apparatus under highly standardized conditions. However, the predictive value of the in vitro dissolution profile with regard to the variability in the individual in vivo dissolution characteristics is often poor. For this reason, many attempts have been made to adapt in vitro dissolution testing to better mimic physiological conditions in order to improve the predictability of in vivo dissolution of API from a dosage form. Alterations were aimed at modifying the temperature and the composition of the dissolution testing media, specifically the pH, ionic strength, buffer capacity as well as the presence of surfactants such as bile salts and/or the presence of digestive enzymes (e.g., pancreatic enzymes), which may greatly influence the characteristics of release from immediate release and extended release dosage forms. However, for conducting dissolution testing with API of low solubility and being formulated in the extended release dosage form, additional improvements are necessary to further tune the testing conditions in order to achieve sufficient in vivo predictability.

It was recognized that API with low solubility can not be instantly released from the extended release dosage form comprising a hydrophilic matrix when exposed to the aqueous media.

The reason is that hydrophilic matrix forms a polymeric gel layer immediately after the dosage form gets in contact with the surrounding media, which in turn prevents the API to be released to the media. The API is released considerably only after the swollen gel layer erodes. Clearly the polymer matrix erosion contributes substantially to overall release kinetics. In vivo erosion of the dosage form is affected by different hydrodynamic conditions, shear stress and friction forces in gastrointestinal tract. Due to difficulties in mimicking the gastrointestinal in vivo conditions and interplay of various factors there is persistent need for new and simplified dissolution techniques enabling good in vivo predictability.

Several concepts for testing dissolution of APIs are known in prior art.

The application of simulated gastrointestinal fluids, also defined as 'biorelevant dissolution media', has gained increasing attention in pharmacopeial monographs including the United States Pharmacopeia.

Kazuhiro Sakoa et al. in Influence of water soluble fillers in hydroxypropylmethylcellulose matrices on in vitro and in vivo drug release, published in the Journal of Controlled Release volume 81, in 2002 on pages 165 to 172 describe broking matrices by spatula to dissolve the matrices in order to correct the 100% drug dissolution. Further it was disclosed that the mechanical stress was given to the matrices by removing the tablets from dissolution media at 1 h after starting the test and transferring the tablets into a 50-ml test-tube with 60 g of glass beads (diameter, 4 mm; density, 2.5 g/cm) and 2 ml of water. The tube was closed, with glass beads and the swollen matrix, turned side ways and the mechanical stress artificially given by shaking the tube horizontally with a shaker. After shaking, the whole content in the tube was returned into the dissolution medium, and the in vitro dissolution test was resumed.

Ian Borst et al. in the article New and extended applications for USP drug release apparatus 3 published in Dissolution technologies in February 1997 disclosed adding inert beads of mixed density.

GB 2 136 123 A describes a dissolution testing apparatus and method using a reciprocating cylinder, wherein a holder with pellets is moved up and down a container with liquid causing the liquid to be flushed in and out of the holder and producing dissolution of the pellets.

S. Aoki et al., "Determination of the mechanical impact force in the in vitro dissolution test and evaluation of the correlation between in vivo and in vitro release", in International Journal of Pharmaceutics, volume 95, pages 67-75, 1993, determined the mechanical impact force in a so-called "paddle-beads method", in which polystyrene beads having a 'specific gravity' of 1.05 $g/cm^3$ were added to the dissolution test medium and the mechanical impact by varying the number of such beads was evaluated.

Y. Machida et al., "A New Method of Dissolution Testing for Oily Drug Preparations Using an Improved Apparatus", in Chemical and Pharmaceutical Bulletin, volume 34, pages 2637-2641, 1986, described a modified version of the paddle method, comprising the addition of polypropylene beads to the test medium in order to enhance the efficiency of stirring.

However, when it comes to dissolution testing of poorly soluble APIs, the above indicated prior art apparatus or processes for dissolution testing of APIs frequently fail to properly predict the in vivo performance.

Therefore, according to the object of the present invention, there is a need for an improved concept for testing dissolution of APIs. The present inventive dissolution testing apparatus and process enable dissolution testing of API from various dosage forms with good in vivo predictability.

Summary of the invention

The present invention provides an apparatus for testing dissolution of an active pharmaceutical ingredient capable of reciprocating a dosage form up and down in a testing media in combination with beads comprising a density of 1.1 g/ml to 1.5 g/ml and capable of inducing mechanical stress to the dosage form while the apparatus is in operation.

The present invention further provides a use of beads comprising a density of 1.1 g/ml to 1.5 g/ml for testing dissolution of an active pharmaceutical ingredient from a dosage form.

Furthermore the present invention provides a use of beads comprising a density of 1.1 g/ml to 1.5 g/ml in combination with an apparatus for testing dissolution of an active pharmaceutical ingredient capable of reciprocating a dosage form up and down in a testing media for testing dissolution of an active pharmaceutical ingredient from a dosage form.

The present invention also provides a process for testing dissolution of an active pharmaceutical ingredient comprising reciprocating a dosage form comprising the active pharmaceutical ingredient and beads comprising a density of 1.1 g/ml to 1.5 g/ml up and down in a testing media.

The present invention yet further provides a process for obtaining a dosage form comprising an active pharmaceutical ingredient with desired dissolution, comprising steps of:

a) providing at least one dosage form comprising the active pharmaceutical ingredient of predetermined composition and/or form,
b) subjecting the at least one dosage form comprising the active pharmaceutical ingredient to a process according to any one of claims 1 to 4, and
c) selecting the dosage form comprising an active pharmaceutical ingredient with desired dissolution,
or
d) providing at least one other dosage form comprising the active pharmaceutical ingredient of predetermined composition and/or form, wherein the at least one other dosage form comprising the active pharmaceutical ingredient vary in composition and/or form from the at least one dosage form comprising active pharmaceutical ingredient, and
e) subjecting the at least one other dosage form comprising the active pharmaceutical ingredient to a process according to any one of claims 1 to 4, and
f) selecting the dosage form comprising an active pharmaceutical ingredient with desired dissolution.

Furthermore, the present invention provides a process for assaying or predicting in vivo dissolution of an active pharmaceutical ingredient, wherein a dissolution of the active pharmaceutical ingredient while employing process according to any one of claims 1 to 4, 13 or 14 is measured and/or determined.

Detailed description of the invention

According to the present invention, it was surprisingly found that realistic physiological conditions of stomach or intestine can be simulated by simple means, even under conditions wherein an API has a poor solubility. Therefore, an exceptional reliable prediction of in vivo dissolution for matrix dosage form comprising API of low solubility is provided. Furthermore, additional advantages over prior art are given. For example, an advantage of the concept of the present invention comprising beads of a density of 1.1 g/ml to 1.5 g/ml within the dissolution apparatus that is capable of reciprocating the dosage form up and down is that the dosage form may be exposed to physical stress during dissolution testing without the need to transfer the dosage form manually from the dissolution testing apparatus to other device that is specifically designed to disturb the structure of the dosage form. Furthermore, the stress forces can be imposed on the dosage form continuously during dissolution testing without interfering with sampling or sampling interval. Said improvement facilitates dissolution of the API from the dosage form in vitro that is commensurate to dissolution in vivo without having to deal with imperfections as for example incomplete dissolution, unnecessary measurement errors or not linear sampling intervals. Besides, said simple means for simulating interactions with moving solid particles of food or other gastrointestinal mechanical destructive forces enable particularly efficient friction between the dosage form and the beads, as the density of beads lower than 1.1 g/ml to 1.5 g/ml leads to floating of beads and the density higher than 1.1 g/ml to 1.5 g/ml makes the beads sink. In either case, the lower or higher density lead to unsatisfactory movement of beads during the up-and-down strokes of the apparatus reducing the number of collisions with the dosage form and thus having no significant effect to the dissolution of the dosage form. Furthermore, the best effect can be achieved when the dosage form being tested is extended release matrix tablet. Mechanical stress induced by the beads of said density causes the extended release matrix tablet to erode faster and prevents API to be trapped in the swollen polymer gel layer. This in turn leads to faster API release, which is more comparable to the situation in the gastrointestinal (GI) tract. Further advantageous is to apply the present invention in cases where dissolution of API with low solubility needs to be studied.

Conventionally in a dissolution apparatus comprising an inner tube, like for example a glass cylinder, e.g. apparatus 3 according to the US Pharmacopeiea USP 31 or any similar dissolution apparatus that comprise an outer tube which contain the testing media, an inner tube where normally the dosage form to be analyzed is placed, wherein the top and base of the inner tube are closed with the mesh, and operation of the apparatus involves various rates of dips of inner tube per minute, namely different velocities of up and down movement of the inner tube inside the outer one, wherein the dosage form gets suspended in the testing media during the reciprocating action; dissolution rate of the API will be affected by the agitation and composition of the dissolution medium. However, the solubility of poorly soluble APIs in *in vivo* relevant dissolution media, such as those described in corresponding documents (e.g. E. Nicolaides et al., Pharmaceutical Research, Vol. 16, No. 12, 1999, p. 1876-1882; J.B. Dressman et al., European Journal of Pharmaceutical Sciences 11 Suppl. 2 (2000), p. 73-80; V.H. Sunesen et al., European Journal of Pharmaceutical Sciences 24 (2005), p. 305-313), where pH, ionic strength or amount of surfactant is varied, is very often too low to obtain useful dissolution data that can be correlated to *in vivo* data. The solubility of the tested API can be increased by the addition of higher concentrations of surfactants or by the addition of organic solvents. However, in this case, the dissolution medium does not reflect the *in vivo* conditions in the intestinal tract and can not resemble mechanical forces of GI tract induced on the dosage form, which very often leads to poor *in vivo* predictability. In addition, varying pH, ionic strength or increasing the concentration of surfactants avail even less when dissolution of low soluble API from the extended release matrix dosage form is to be determined. The present invention remedies said imperfection and provides simple means to induce mechanical forces to the dosage form during dissolution testing.

Active pharmaceutical ingredient (API) denotes according to the present invention any drug that can be used for treatment or prophylaxis in animal, particularly in a mammal, specifically in human. According to any embodiment of the present invention, the active pharmaceutical ingredient is selected from the group of analgesics, antiinflammatory agents, anthelmintics, anti-arrhythmic agents, anti-asthma agents, antibacterial agents, anti-viral agents, anticoagulants, anti-depressants, anti-diabetics, antiepileptics, anti-fungal agents, anti-gout agents, anti-hypertensive agents, anti-malarials, anti-migraine agents, anti-muscarinic agents, antineoplastic agents and immunosuppressants, anti-protozoal agents, anti-thyroid agents, anti-tussives, anxiolytic, sedatives, hypnotics, neuroleptics, β-blockers, cardiac inotropic agents, corticosteroids, diuretics, anti-parkinsonian agents, gastro-intestinal agents, histamine-receptor antagonists, keratolytics, lipid regulating agents, muscle relaxants, anti-anginal agents, nutritional agents, analgesics, sex hormones, stimulants, peptides, peptidomimetics, DNA, RNA, oligodeoxynucleotides, genetic material, proteins, oligonucleotides, and vaccines. Best effect according to the present invention can be observed when the API has low solubility, namely has solubility of less than 0,5 mg/ml, preferably less than 0.1, in a pH range from 1 to 7.5 at 37°C. Solubility is measured in aqueous solvent. In this way, the present invention provides reliable correlation between in vivo and in vitro dissolution even with compounds having low solubility.

Dosage form according to the present invention is understood as any single or multi unit solid pharmaceutical formulation, such as for example tablet or capsule, preferably a tablet. In particular, the dosage form indicates herein extended release matrix pharmaceutical formulation, wherein said formulation comprises a hydrophilic polymer in at least one layer of the pharmaceutical formulation, i.e. in the coating of the coated tablet, a tablet layer of the bilayer tablet or just in a tablet as such, and at least 80 % of the API is dissolved in more than 60 minutes, preferably 2 hours, under the following conditions: using a USP Apparatus 2 placing the pharmaceutical formulation in 900 ml of media that enables sink conditions at 37°C and 75 rpm and measuring the amount of API dissolved at 1 hour. The progress of dissolution may also be monitored at various time points. The skilled person would know how to take a sample and determine the dissolved amount of the API. Techniques such as HPLC or UV spectroscopy can be applied. The term "dissolution" as used herein refers to dissolution in the intestine comprising the steps of disintegration of an API or a pharmaceutical formulation comprising the API and following solubilisation of the API. Hydrophilic polymer used in the dosage form can be any polymer whose hydration results in formation of the gel layer that controls the release rate of the API. As a rule, the swollen hydrophilic polymer prevents the API to be released from the matrix. Instead the polymer must erode first to expose the API to the media, which solubilizes and dissolves the API. Preferred hydrophilic polymers are cellulose or cellulose derivatives such as carboxymethylcellulose sodium, hydroxyethyl cellulose, hydroxypropyl cellulose, hydroxypropyl methylcellulose, or methylcellulose; polyethylene glycol, polyvinylalcohol, alginic acid or its salts, gelatine, starch or pregelatinized starch, xanthan gum, galactomannans and glucomannans, Karaya gum, chitosan, carbopol, carrageenans and other natural gums or resins. Hydrophilic polymer can comprise 2 % to 80 % (by weight) of the final dosage form, preferably 2% to 60 %, more preferably 10 % to 50 %. Particularly the present invention provides the apparatus, process and use, where the dosage form comprises cellulose or cellulose derivatives, or polyethylene glycol. Dosage forms in said embodiments comprising carboxymethylcellulose sodium, hydroxyethyl cellulose, hydroxypropyl cellulose, hydroxypropyl methylcellulose, or methylcellulose, specifically hydroxypropyl methylcellulose are embraced with the present invention.

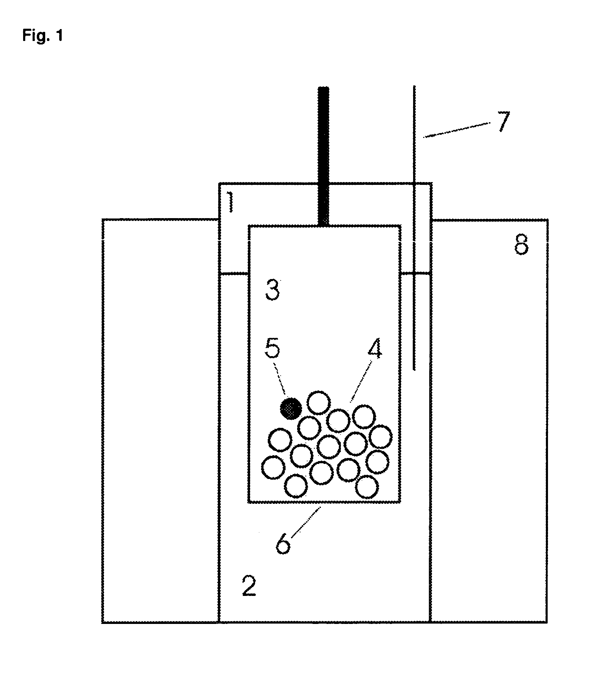

The present invention relates to an apparatus for testing dissolution of an active pharmaceutical ingredient capable of reciprocating a dosage form up and down in a testing media in combination with beads comprising a density of 1.1 g/ml to 1.5 g/ml and capable of inducing mechanical stress to the dosage form while the apparatus is in operation. Particularly the present invention relates to an apparatus for testing dissolution of an active pharmaceutical ingredient comprising an outer compartment (1), inner compartment (3), wherein inner compartment has at least a base (6) perforated, preferably top and the base, and the inner compartment can be reciprocated up and down inside the outer compartment, in combination with the beads comprising a density of 1.1 g/ml to 1.5 g/ml (4). Said beads are for use in the inner compartment. When the apparatus is in operation, the apparatus further comprises testing media (2), wherein testing media is in the outer compartment and at least repeatedly in the inner compartment, a dosage form (5), wherein the dosage form and beads are placed in the inner compartment and beads are capable of inducing mechanical stress to the dosage form, means for taking a sample (7) like for example a sampling probe and means for regulating the temperature of the compartments with the testing media (8), like for example a water bath. The inner (3) or outer (1) compartment can be for example a cylinder made of glass. The perforated base (6) of the inner compartment is preferably a mesh. While the apparatus is in operation, the dosage form and beads are at least repeatedly in the testing media. Means for regulating the temperature (8) can be set at the specific temperature, which in turn leads to setting the temperature of the testing media to the substantially the same temperature, preferably the same temperature. Temperature can be set at physiological temperatures of a mammal, preferably between 25 and 42°C, more preferably between 35 to 40°C and most preferably about 37°C. Schematic presentation of the apparatus is depicted in Fig. 1. The present apparatus may comprise the beads that comprise balls with diameter of at least 4 mm. Said diameter was proved advantageous as it causes beads not to get caught in the gel layer of the swollen dosage form. Instead, the beads remain freely moving and hit the dosage form during the reciprocating movement and thus act abrasively to the gel layer. On the other hand, beads with the diameter of less than 4 mm tend to stick to the gel layer and defend the dosage form from further hits of other beads. Thus, the continuous mechanical stress to the dosage form is reduced and in vivo correlation diminished. Also advantageous is when the beads are at least 25 % of a size of the dosage form since it enables to impose significant physical force to the dosage form. The specific embodiments provide for a more realistic simulation of physiological conditions in view of physical factors in GI motility and temperature conditions within a body. Thus, these means further contribute to an improved correlation between in vivo and in vitro dissolution. According to another embodiment the apparatus further comprises means for mixing a testing media. This feature leads to uniform distribution of the released API in the testing media and thus reduces sampling errors. Specific embodiment of the present invention is use of beads comprising a density of 1.1 g/ml to 1.5 g/ml in combination with an apparatus for testing dissolution of an active pharmaceutical ingredient capable of reciprocating a dosage form up and down in a testing media for testing dissolution of an active pharmaceutical ingredient from a dosage form.

The aforementioned apparatus in combination with said beads is particularly suitable for testing dissolution of an active pharmaceutical ingredient from a dosage form. Use of the aforementioned apparatus in combination with said beads for testing dissolution of an active pharmaceutical ingredient from a dosage form is encompassed within the present invention.

The "beads" employed in the apparatus, process or use of the present invention is a group of small balls, wherein each of the ball is of density of 1.1 g/ml to 1.5 g/ml. Beads can be made of metal, glass, natural material such as for example rubber or latex, or synthetic plastic material. It is understood that in cases where the material originally has the density different of 1.1 g/ml to 1.5 g/ml, it must be either compacted to said density or perforated or made hollow. Preferably polymers and plastic materials are used for manufacture of the beads. Examples include acetal, acrylonitrile-butadiene-styrene (ABS), acrylic, bismaleimide (BMI), cellulosic, epoxy (EP), ethylene copolymer, fluoropolymer, ionomer, and ketones such as polyaryletherketone and polyetheretherketone (PEEK), polyamide, polyimide, polybutadiene, polycarbonate (PC), polyethylene, polyethylene terphthalate (PET), and polybutylene terphthalate (PBT), polyester, vinyl ester, polyether block amide (PEBA) resins, polyetherimide, polyolefin, polyphenylene oxide (PPO), polypropylene (PP), polysulphide, polysulphone, or polyphthalamide, silicone, styrene, styrene copolymer, and vinyl. The manufacturing of the beads can for example comprise molding of material in the ball shape or can involve hot melting, pressing, film drying, thermosetting or cross-linking, room-temperature curing or vulcanizing, solidification with ultraviolet light (UV). Present invention also relates to a use of beads comprising a density of 1.1 g/ml to 1.5 g/ml for testing dissolution of an active pharmaceutical ingredient from a dosage form. Particularly useful are beads for testing dissolution of API when the dosage form is an extended release matrix pharmaceutical formulation. Specifically it is useful in cases where API in an extended release matrix pharmaceutical formulation has solubility of less than 0,5 mg/ml, preferably less than 0.1, in a pH range from 1 to 7.5 at 37°C.

The present invention also relates the a process for testing dissolution of an active pharmaceutical ingredient comprising reciprocating a dosage form comprising the active pharmaceutical ingredient and beads comprising a density of 1.1 g/ml to 1.5 g/ml up and down in a testing media. Preferably, the process involves using the aforementioned apparatus, like for example wherein the beads comprise balls with diameter of at least 4 mm or wherein the beads are at least 25 % of a size of the dosage form. The best result is achieved when the dosage form is a extended release matrix pharmaceutical formulation such as tablet. The mass of beads used in the process can range from 4 g to 20 g, preferably from 5 g to 15 g, more preferably from 8 g to 12 g, specifically is 8 g.

The mechanical stress according to the present invention is inevitably put on the dosage form and thus process can be employed without removing the dosage form from the apparatus. This facilitates continuous process and enhances robustness and automatization. In addition, the process enables to induce mechanical stress throughout the dissolution test until the dosage form is completely disintegrated. However, it is understood that the dosage form can be transferred from one compartment to the other same compartment in order to perform the process in different testing media.

According to this further aspect of the invention, a reliably screening for an active pharmaceutical ingredient in predetermined condition or form having the desired dissolution properties is provided. Thereby, the number of extensive and costly *in vivo* dissolution tests in order to find the pharmaceutical composition having the desired dissolution properties can be significantly reduced. Furthermore, the invention provides for an process for obtaining a dosage form comprising an active pharmaceutical ingredient with desired dissolution, comprising steps of:

a) providing at least one dosage form comprising the active pharmaceutical ingredient of predetermined composition and/or form,
b) subjecting the at least one dosage form comprising the active pharmaceutical ingredient to a process according to any of above embodiments, and
c) selecting the dosage form comprising an active pharmaceutical ingredient with desired dissolution,
or
d) providing at least one other dosage form comprising the active pharmaceutical ingredient of predetermined composition and/or form, wherein the at least one other dosage form comprising the active pharmaceutical ingredient vary in composition and/or form from the at least one dosage form comprising active pharmaceutical ingredient, and
e) subjecting the at least one other dosage form comprising the active pharmaceutical ingredient to a process according to any of above embodiments, and
f) selecting the dosage form comprising an active pharmaceutical ingredient with desired dissolution.

The composition and/or form used in the process for obtaining the dosage form comprising with desired dissolution vary in pharmaceutically acceptable excipients, amount, particle size and/or polymorphic form, bulk density, charge of the said, technology used for preparing the dosage form (i.e. compression degree). Also the condition of the active pharmaceutical ingredient in the process can be selected from particle size, polymorphic forms, bulk density, charge or the route of synthesis of pharmaceutical active ingredient. The skilled person will understand how to interpret dissolution data and know how to apply pharmaceutical technology or chemical synthesis techniques in the art to properly modify the composition and/or form in order to arrive at the one with the desired dissolution. In every embodiment relating to the testing dissolution of the API the sample volumes can be taken at desired time points and analytically analyzed for the amount of API comprised in each sample volume.

It is understood that the selection of the testing medium strongly depends on the nature of the API to be tested. To achieve significant in vivo relevant dissolution testing various dissolution media can be used in combination with beads. Testing media chosen is desirably simulating physiological fluids or buffer systems. Preferably the pH of the testing medium is in a range of pH 1.0 to pH 5.0 simulating conditions in the stomach, about pH 5.0 to 8.0, preferably 6.0 to colon. Testing media is normally a buffer solution comprising salts like for example NaCl, KCl, $CaCl_2$, bile salts (e.g. sodium taurocholate), phospholipids (e.g. lecithin), organic acid (e.g. maleic acid), fatty acid (e.g. sodium oleate) or esters of fatty acid (e.g. glyceryl monooleate). Preferably the testing media comprises bile salts. The bile salts can be present in a concentration range of 0.1 to 50 mM. Particularly, the testing media comprises phospholipids, preferably lecithin. The phospholipids can be present in a concentration range of 0.1 to 10 mM. The specific buffer solution can be used as a testing media in the process as described above to mimic stomach in a fed or fasted state. For example media like simulated gastric fluid, fasted state simulated intestinal fluid (FaSSIF), fed state simulated intestinal fluid (FeSSIF) and other physiological relevant media can be used. To further reflect the physiological conditions in the body the testing media can be replaced or changed during dissolution testing process in order to achieve sink condition. "Sink condition" describes a dissolution system wherein the concentration of API is sufficiently dilute so that the dissolution process is not impeded by approach to saturation of the compound of interest, that is the API. Typically, sink conditions are considered to exist if the volume of dissolution media is equal to or greater than three times necessary to make a saturated solution of the API. Under a sink condition, dissolution of the API is not limited by, or determined by the solubility in the dissolution medium. However, for poorly soluble APIs, sink conditions do not occur *in vivo.* The concentration of API in the intestinal tract will reach saturation, and the dissolution rate will then depend on the rate of absorption of the active substance across the intestinal epithelium into the intestinal blood vessels.

Brief description of the Drawing

Fig. 1 shows a schematic drawing of a dissolution apparatus according to an embodiment of the invention. Fig. 1 depicts the general constitution of the present apparatus for dissolution testing according to an embodiment of the present invention, which comprises an outer compartment (1), inner compartment (3), wherein the inner compartment can be reciprocated up and down inside the outer compartment and the inner compartment has at least a base (6) perforated, preferably top and the base, in combination with the beads comprising a density of 1.1 g/ml to 1.5 g/ml (4). When the apparatus is in operation, the apparatus further comprises testing media (2), wherein testing media is in the outer compartment and in the inner compartment at least while the said is being reciprocated, a dosage form (5), wherein the dosage form and beads are placed in the inner compartment and beads, means for taking a sample (7) like for example a sampling probe and means for regulating the temperature of the compartments with the testing media (8), like for example a water bath.

Fig. 2 cumulative amount of drug released (%) from formulations A and B plotted versus time (h); tested with beads dissolution system (USP apparatus 3 in combination with beads of density ranging from 1.1 g/ml to 1.5 g/ml), program: 1 h/ 20 DPM, 15 min/40 DPM, remaining testing time in 25 DPM; media: water.

Fig. 3 cumulative amount of drug released (%) from formulations A and B plotted versus time (h); tested with beads dissolution system (USP apparatus 3 in combination with beads of density ranging from 1.1 g/ml to 1.5 g/ml), program: 1 h/ 20 DPM, 15 min/40 DPM, remaining testing time in 25 DPM; media: hydrogen carbonate buffer media pH 6.5 + 0.27% NaCl + 0.07% KCl + 0.005% $CaCl_2$

Fig. 4 cumulative amount of drug released (%) from formulations A and B plotted versus time (h); tested with USP Apparatus 1 (basket method); 100 rpm, media: phosphate buffer, pH 6.8

Fig. 5 cumulative amount of drug released (%) from formulations A and B plotted versus time (h); tested with USP Apparatus 1 (basket method); 150 rpm, media: water + 0.8% NaCl + 0.04% KCl

Fig. 6 cumulative amount of drug released (%) from formulations A and B plotted versus time (h); tested with USP Apparatus 2 (paddle method); 100 rpm, tablet in sinker, media: water.

Fig. 7 cumulative amount of drug released (%) from formulations A and B plotted versus time (h); tested with USP Apparatus 3 (reciprocating inner tube); 1 h/ 20 DPM, 15 min/40 DPM, remaining test time in 25 DPM, media: water

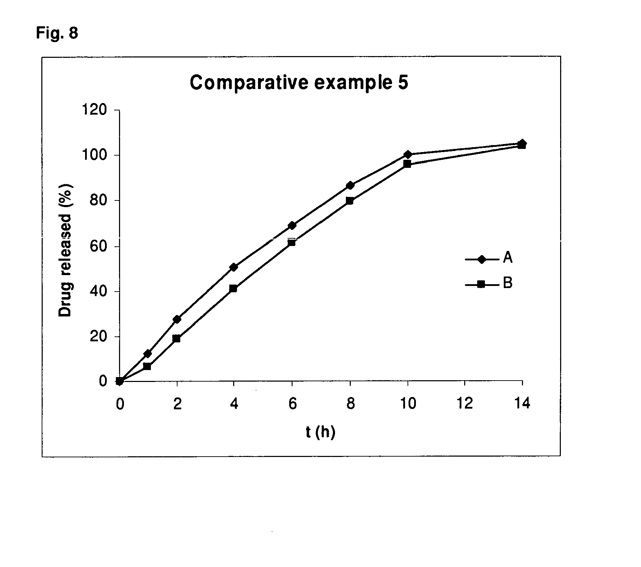

Fig. 8 cumulative amount of drug released (%) from formulations A and B plotted versus time (h); tested with USP Apparatus 3 (reciprocating inner tube); 1 h/ 20 DPM, 15 min/40 DPM, remaining test time in 25 DPM; media: hydrogen carbonate buffer, pH 6.5 + 0.27% NaCl + 0.07% KCl + 0.005% $CaCl_2$.

The following examples are merely illustrative of the present invention and it should not be considered as limiting the scope of the invention in any way. The examples and modifications or other equivalents thereof will become apparent to those versed in the art in the light of the present entire disclosure.

Example

Two hydrophilic hydroxypropyl methylcellulose (HMPC) matrix tablet formulations (diameter 8 mm) containing substance with pH independent solubility in water of less than 0.1 mg/ml, pKa 8.8 (weak acid) (formulation A and formulation B) were tested in human relative bioavailability study under fasting conditions. The study was conducted in 26 healthy volunteers in an open-label, randomized, crossover, single dose study design. Both formulations were tested for $C_{max}$ and AUC parameters, ratio A/B for both parameters was calculated. The results for $C_{max}$ ratio A/B was 122,3% and $AUC_t$ A/B ratio was 104,2%. High $C_{max}$ ratio was not reflected with conventional *in vitro* dissolution testing, therefore new method needed to be developed.

In the examples and comparable examples below the results are presented from testing the tablets with apparatus comprising the beads of density of 1.1 g/ml to 1.5 g/ml and with apparatus without said beads, respectively.

Example 1

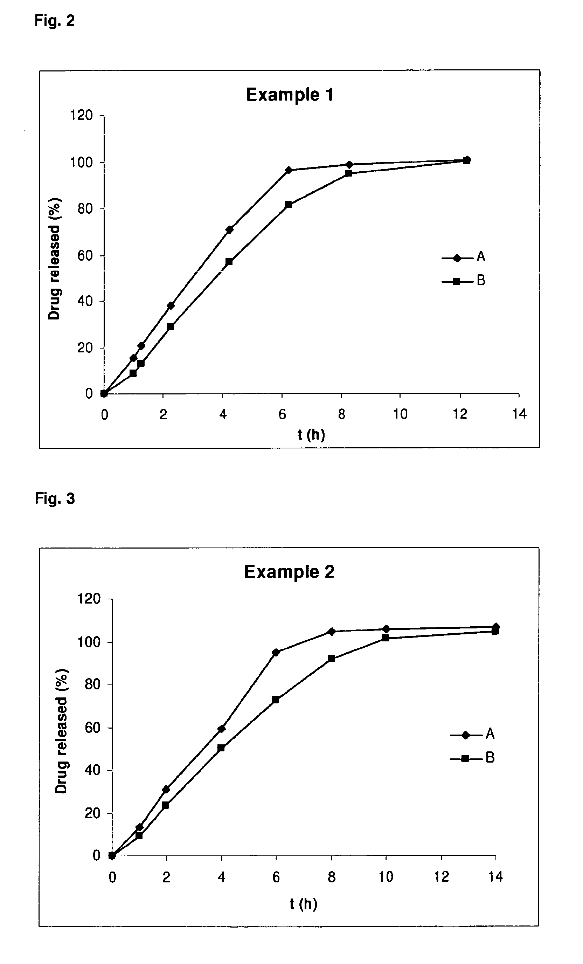

The dissolution test procedure using beads was performed as follows: Testing media, which was water, was degassed and 250 ml was poured into each dissolution vessel. Dissolution vessels were placed in water bath to maintain temperature 37°C. 8 g of beads (diameter 8 mm) were weighed and placed into inner compartment (3). Tablets A and B were weighed and placed on top of beads. The dissolution apparatus settings were 1 hour: 20 DPM, 15 min: 40 DPM and the remaining part of the test with 25 DPM. The middle 15 min phase simulates the gastric emptying phase. Sample time points were 1, 2, 4, 6, 8, 10 and 14 hours. After the samples were automatically collected they were analyzed on HPLC system and cumulative amount of the dissolved drug was calculated. Results are depicted in Fig. 2.

Example 2

The dissolution test procedure using beads was performed as in example 1, only that hydrogen carbonate buffer media pH 6.5, further comprising 0.27% NaCl, 0.07% KCl and 0.005% CaCl$_2$ was used as a testing media. Results (cumulative amount of drug released (%) from formulations A and B plotted versus time (h)) are depicted in Fig. 3.

Comparative example 1

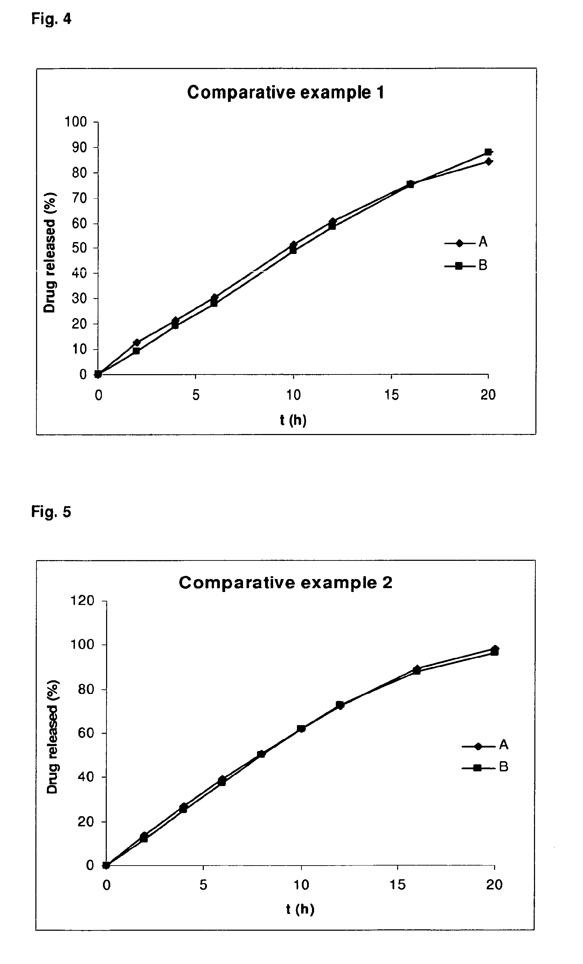

Dissolution test procedure was performed on the USP apparatus 1 (basket method) at 100 rpm in a phosphate buffer (pH 6.8). Sample time points were again 1, 2, 4, 6, 8, 10 and 14 hours. After the samples were automatically collected they were analyzed on HPLC system and cumulative amount of the dissolved drug was calculated. Results are presented in Fig. 4.

Comparative example 2

Comparative example 2 was performed in the same way as in comparative example 1, only that the agitation was set at 150 rpm and the testing media used was water with 0.8% NaCl and 0.04% KCl. Results are shown in Fig. 5.

Comparative example 3

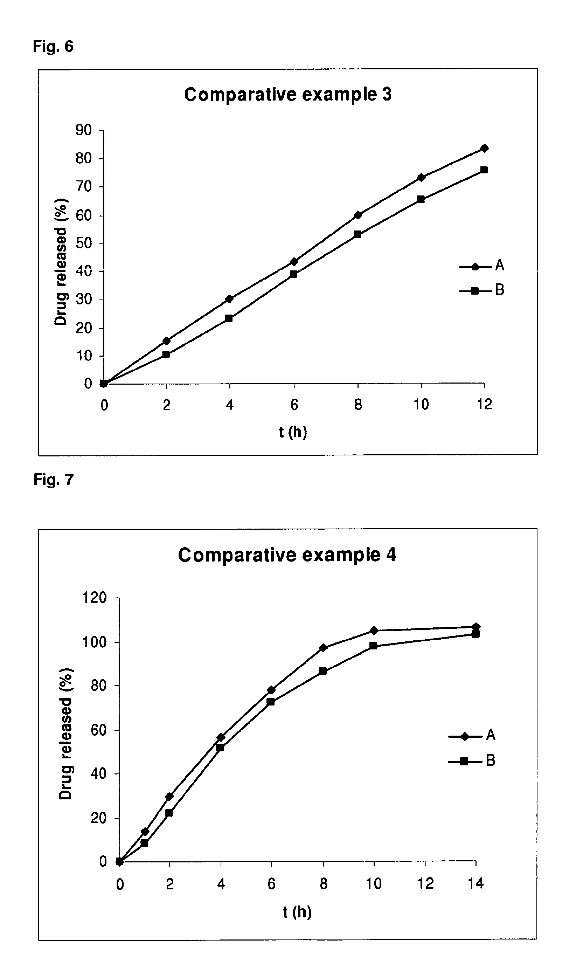

In comparable example 3 the dosage forms were tested by using USP Apparatus 2 (paddle method) set at 100 rpm. Tablet was placed in a sinker to avoid sticking and to prevent floating of the tablet in the media after the gel layer is formed. The testing media was water. Results are presented in Fig. 6.

Comparative example 4:

Apparatus 3 (reciprocating inner tube) was used to test dissolution of the API from the formulations A and B. Agitation rate was set at 1 h at 20 DPM, 15 min at 40 DPM and the remaining test time at 25 DPM. The testing media was water. Cumulative amount of drug released (%) from formulations A and B plotted versus time (h) is depicted in Fig. 7.

Comparative example 5:

Test was performed as in comparative example 4. Testing media was hydrogen carbonate buffer, pH 6.5, supplemented with 0.27% NaCl, 0.07% KCl and 0.005% CaCl$_2$. Results are shown in Fig. 6.

Examples 1 and 2 show better discriminatory power of beads dissolution system than conventional dissolution tests shown in Comparative examples 1 to 5. Tests in examples 1 and 2 are also in good agreement with *in vivo* data.

The dissolution system described in test Example 1 was used to develop the *in vitro-in vivo* correlation model for the tested extended release matrix tablet using Gastro Plus simulation software. A good linear correlation between the fraction of the absorbed drug in vivo and in vitro was observed with the r (linear regression coefficient) = 0.925. Based on this model the maximal plasma concentration (Cmax) was predicted and compared with the observed values. The percent prediction error (%PE) was calculated according:

$$\%PE = ((observed\ value - predicted\ value)/observed\ value) \times 100$$

The average absolute percent prediction error was 7.3% (N=4) and the %PE for each tested matrix tablet was less than 15%, which demonstrates a good internal predictability of the in vivo in vitro correlation.

No such good in vivo in vitro correlation can be obtained using the conventional dissolution methods without using beads of specific density, which is evident from Comparative examples 1, 2, 3, 4 and 5.

In the above example, dissolution testing of the model drug is described. As should become apparent, the present invention can be applied for dissolution testing of other APIs formulated in different dosage forms. When testing other APIs, a testing media different from the described may be used, e.g. a relatively strong acidic aqueous media, a media comprising serum albumins or other means for chemically or physically improving solubility of the API to be tested. Also, various agitation rates can be applied. Furthermore, it is understood that the composition of the testing media will depend on the intestine conditions, for example fasted state or fed state, to be tested. Furthermore, the amount (mass) of beads used can depend on the size or shape of the formulation tested. The same is relevant for the diameter of beads used.

**Claims**

1.  A process for testing dissolution of an active pharmaceutical ingredient comprising reciprocating a dosage form comprising the active pharmaceutical ingredient and beads comprising a density of 1.1 g/ml to 1.5 g/ml up and down in a testing media.

2.  The process for testing dissolution of an active pharmaceutical ingredient according to claim 1, wherein the dosage form is an extended release matrix pharmaceutical formulation.

3.  The process according to claim 1 or 2, wherein the beads are at least 25 % of a size of the dosage form.

4.  The process according to any one of the claims 1 to 3, wherein the beads comprise balls with diameter of at least 4 mm.

5.  An apparatus for testing dissolution of an active pharmaceutical ingredient capable of reciprocating a dosage form (5) up and down in a testing media (2) in combination with beads (4) comprising a density of 1.1 g/ml to 1.5 g/ml and capable of inducing mechanical stress to the dosage form while the apparatus is in operation.

6.  The apparatus according to claim 5 for testing dissolution of an active pharmaceutical ingredient capable of reciprocating a dosage form up and down in a testing media in combination with beads wherein the dosage form and beads are in the testing media while the apparatus is in operation.

7.  The apparatus according to claim 5 or 6 for testing dissolution of an active pharmaceutical ingredient capable of reciprocating a dosage form up and down in a testing media in combination with beads, wherein the beads comprise balls with diameter of at least 4 mm.

8.  Use of beads comprising a density of 1.1 g/ml to 1.5 g/ml for testing dissolution of an active pharmaceutical ingredient from a dosage form.

9.  Use of beads according to claim 8, wherein dosage form is an extended release matrix pharmaceutical formulation.

10.  Use of beads according to claim 8 , wherein said beads are used in combination with an apparatus for testing dissolution of an active pharmaceutical ingredient capable of reciprocating a dosage form up and down in a testing media.

11.  A process for obtaining a dosage form comprising an active pharmaceutical ingredient with desired dissolution, comprising steps of:

     a) providing at least one dosage form comprising the active pharmaceutical ingredient of predetermined composition and/or form,
     b) subjecting the at least one dosage form comprising the active pharmaceutical ingredient to a process according to any one of claims 1 to 4, and
     c) selecting the dosage form comprising an active pharmaceutical ingredient with desired dissolution,
     or
     d) providing at least one other dosage form comprising the active pharmaceutical ingredient of predetermined composition and/or form, wherein the at least one other dosage form comprising the active pharmaceutical ingredient vary in composition and/or form from the at least one dosage form comprising active pharmaceutical ingredient, and
     e) subjecting the at least one other dosage form comprising the active pharmaceutical ingredient to a process according to any one of claims 1 to 4, and
     f) selecting the dosage form comprising an active pharmaceutical ingredient with desired dissolution.

12.  The process according to claim 11, wherein the composition and/or form vary in pharmaceutically acceptable excipients, amount, particle size and/or polymorphic form, bulk density, charge of the said, technology used for preparing the dosage form; or load, particle size and/or polymorphic form, density, charge or the route of synthesis of pharmaceutical active ingredient.

13.  The process according to any one of claims 1 to 4, 11 or 12, wherein buffer mimicking a fed stomach is used as a testing media.

**14.** The process according to any one of claims 1 to 4, or 11 to 13, further comprising replacing or changing the testing media during dissolution testing.

**15.** A process for assaying or predicting in vivo dissolution of an active pharmaceutical ingredient, wherein a dissolution of the active pharmaceutical ingredient while employing process according to any one of claims 1 to 4, 13 or 14 is measured and/or determined.

**Patentansprüche**

**1.** Verfahren zum Testen der Auflösung eines Wirkstoffes durch Auf- und Abbewegen einer den Wirkstoff enthaltenden Darreichungsform und Perlen mit einer Dichte von 1,1 g/ml bis 1,5 g/ml in einem Testmedium.

**2.** Verfahren zum Testen der Auflösung eines Wirkstoffes gemäß Anspruch 1, wobei es ich bei der Darreichungsform um eine Retardmatrix-Arzneiformulierung handelt.

**3.** Verfahren gemäß Anspruch 1 oder 2, wobei die Perlen mindestens 25 % der Größe der Darreichungsform entsprechen.

**4.** Verfahren gemäß Anspruch 1 bis 3, wobei es sich bei den Perlen um Kügelchen mit einem Durchmesser von mindestens 4 mm handelt.

**5.** Gerät zum Testen der Auflösung eines Wirkstoffes mit der Fähigkeit, eine Darreichungsform (5) in einem Testmedium (2) zusammen mit Perlen (4) mit einer Dichte von 1,1 g/ml bis 1,5 g/ml auf und ab zu bewegen und der Fähigkeit, eine mechanische Belastung auf die Darreichungsform auszuüben, während das Gerät in Betrieb ist.

**6.** Gerät gemäß Anspruch 5 zum Testen der Auflösung eines Wirkstoffes mit der Fähigkeit, eine Darreichungsform in einem Testmedium zusammen mit Perlen auf und ab zu bewegen, wobei sich die Darreichungsform und die Perlen im Testmedium befinden, während das Gerät in Betrieb ist.

**7.** Gerät gemäß Anspruch 5 oder 6 zum Testen der Auflösung eines Wirkstoffes mit der Fähigkeit, eine Darreichungsform zusammen mit Perlen in einem Testmedium auf und ab zu bewegen, wobei es sich bei den Perlen um Kügelchen mit einem Durchmesser von mindestens 4 mm handelt.

**8.** Verwendung von Perlen mit einer Dichte von 1,1 g/ml bis 1,5 g/ml zum Testen der Auflösung eines Wirkstoffes von einer Darreichungsform.

**9.** Verwendung von Perlen gemäß Anspruch 8, wobei es sich bei der Darreichungsform um eine Retardmatrix-Arzneiformulierung handelt.

**10.** Verwendung von Perlen gemäß Anspruch 8, wobei die besagten Perlen zusammen mit dem Gerät zum Testen der Auflösung eines Wirkstoffes verwendet werden, das die Fähigkeit besitzt, eine Darreichungsform in einem Testmedium auf und ab zu bewegen.

**11.** Verfahren zur Erhaltung einer den Wirkstoff enthaltenden Darreichungsform mit der gewünschten Auflösung, bestehend aus den folgenden Schritten:

a) Bereitstellung mindestens einer einen Wirkstoff enthaltenden Darreichungsform mit einer vorbestimmten Zusammensetzung und/oder Form
b) Aussetzung von mindestens einer den Wirkstoff enthaltenden Darreichungsform einem Verfahren gemäß Anspruch 1 bis 4, und
c) Auswahl der den Wirkstoff enthaltenden Darreichungsform mit der gewünschten Auflösung
oder
d) Bereitstellung von mindestens einer anderen den Wirkstoff enthaltenden Darreichungsform mit vorbestimmter Zusammensetzung und/oder Form, wobei die mindestens eine andere den Wirkstoff enthaltende Darreichungsform in Zusammensetzung und/oder Form von der mindestens einen den Wirkstoff enthaltenden Darreichungsform abweicht und
e) Aussetzung der mindestens einen anderen den Wirkstoff enthaltenden Darreichungsform einem Verfahren gemäß einem der Ansprüche 1 bis 4 und

f) Auswahl der Darreichungsform, die einen Wirkstoff mit der gewünschten Auflösung enthält.

12. Verfahren gemäß Anspruch 11, wobei die Zusammensetzung und/oder Form in Bezug auf pharmazeutisch annehmbare Hilfsstoffe, Menge, Partikelgröße und/oder polymorphe Form, Schüttdichte, Ladung des genannten, Technologie, die zur Zubereitung der Darreichungsform verwendet wurde, bzw. in Bezug auf Beladung, Partikelgröße und/oder polymorphe Form, Dichte, Ladung oder Syntheseweg des Wirkstoffes abweicht.

13. Verfahren gemäß einem der Ansprüche 1 bis 4, 11 oder 12, wobei ein Puffer, der einen gefüllten Magen nachahmt, als Testmedium verwendet wird.

14. Verfahren gemäß einem der Ansprüche 1 bis 4, oder 11 bis 13, wobei außerdem das Testmedium während des Auflösungstests gewechselt wird.

15. Verfahren zum Prüfen oder Vorhersagen der In-vivo-Auflösung eines Wirkstoffes, wobei eine Auflösung des Wirkstoffes während des Durchführens eines Verfahrens gemäß einem der Ansprüche 1 bis 4, 13 oder 14 gemessen und/oder bestimmt wird.

## Revendications

1. Un procédé pour tester la dissolution d'un ingrédient pharmaceutique actif consistant à imprimer un mouvement alternatif de haut en bas à une forme galénique comprenant l'ingrédient pharmaceutique actif et des perles contenant une densité de 1,1 g/ml à 1,5 g/ml dans un milieu d'essai.

2. Le procédé pour tester la dissolution d'un ingrédient pharmaceutique actif selon la revendication 1, où la forme galénique est une formulation pharmaceutique à matrice à libération prolongée.

3. Le procédé selon la revendication 1 ou 2, où les perles font au moins 25 % de la taille de la forme galénique.

4. Le procédé selon l'une quelconque des revendications 1 à 3, où les perles comportent des billes d'au moins 4 mm de diamètre.

5. Un appareil pour tester la dissolution d'un ingrédient pharmaceutique actif capable d'imprimer un mouvement alternatif de haut en bas à une forme galénique (5) dans un milieu d'essai (2) en combinaison avec des perles (4) comprenant une densité de 1,1 g/ml à 1,5 g/ml et capable de provoquer des contraintes mécaniques à la forme galénique lorsque l'appareil est en fonctionnement.

6. L'appareil selon la revendication 5 pour tester la dissolution d'un ingrédient pharmaceutique actif capable d'imprimer un mouvement alternatif de haut en bas à une forme galénique dans un milieu d'essai en combinaison avec des perles, où la forme galénique et les perles sont dans le milieu d'essai lorsque l'appareil est en fonctionnement.

7. L'appareil selon la revendication 5 ou 6 pour tester la dissolution d'un ingrédient pharmaceutique actif capable d'imprimer un mouvement alternatif de haut en bas à une forme galénique dans un milieu d'essai en combinaison avec des perles, où les perles sont composées de billes d'au moins 4 mm de diamètre.

8. Utilisation de perles comprenant une densité de 1,1 g/ml à 1,5 g/ml pour tester la dissolution d'un ingrédient pharmaceutique actif issu d'une forme galénique.

9. Utilisation de perles selon la revendication 8, où la forme galénique est une formulation pharmaceutique à matrice à libération prolongée.

10. Utilisation de perles selon la revendication 8, où lesdites perles sont utilisées en combinaison avec un appareil pour tester la dissolution d'un ingrédient pharmaceutique actif capable d'imprimer un mouvement alternatif de haut en bas à une forme galénique dans un milieu d'essai.

11. Un procédé pour obtenir une forme galénique comprenant un ingrédient pharmaceutique actif ayant une dissolution désirée, comportant les étapes suivantes :

a) apporter au moins une forme galénique comprenant l'ingrédient pharmaceutique actif de composition et/ou de forme prédéterminées,

b) soumettre l'au moins une forme galénique comprenant l'ingrédient pharmaceutique actif au procédé selon l'une quelconque des revendications 1 à 4, et

c) sélectionner la forme galénique comprenant un ingrédient pharmaceutique actif ayant la dissolution désirée, ou

d) apporter au moins une autre forme galénique contenant l'ingrédient pharmaceutique actif de composition et/ou de forme prédéterminées, où l'au moins une autre forme galénique comprenant l'ingrédient pharmaceutique actif varie en composition et/ou en forme par rapport à l'au moins une forme galénique comprenant l'ingrédient pharmaceutique actif, et

e) soumettre l'au moins une autre forme galénique comprenant l'ingrédient pharmaceutique actif au ,procédé selon l'une quelconque des revendications 1 à 4,
et

f) sélectionner la forme galénique comprenant un ingrédient pharmaceutique actif ayant la dissolution désirée.

12. Le procédé selon la revendication 11, où la composition et/ou la forme varient de façon pharmaceutiquement acceptable en termes d'excipients, de quantité, de taille de particules et/ou de forme polymorphe, de densité apparente, de charge de celle-ci, de technologie utilisée pour préparer la forme galénique ; ou en termes de charge, de taille de particules et/ou de forme polymorphe, de densité, de poids ou de voie de synthèse de l'ingrédient pharmaceutique actif.

13. Le procédé selon l'une quelconque des revendications 1 à 4, 11 ou 12, où une solution tampon simulant un estomac alimenté est utilisé comme milieu d'essai.

14. Le procédé selon l'une quelconque des revendications 1 à 4, 11 ou 13, comprenant, remplaçant ou changeant le milieu d'essai au cours de l'essai de dissolution.

15. Un procédé d'essai ou de prévision in vivo de la dissolution d'un ingrédient pharmaceutique actif, où une dissolution de l'ingrédient pharmaceutique actif est mesurée et/ou déterminée lors de l'utilisation du procédé selon l'une quelconque des revendications 1 à 4, 13 ou 14.

**Fig. 1**

Fig. 2

Fig. 3

**Fig. 4**

Comparative example 1

**Fig. 5**

Comparative example 2

**Fig. 6**

Comparative example 3

**Fig. 7**

Comparative example 4

**Fig. 8**

Comparative example 5

Drug released (%) vs t (h)

Legend: ◆ A, ■ B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 2136123 A

### Non-patent literature cited in the description

- **KAZUHIRO SAKOA et al.** Influence of water soluble fillers in hydroxypropylmethylcellulose matrices on in vitro and in vivo drug release. *Journal of Controlled Release,* 2002, vol. 81, 165-172
- **S. AOKI et al.** Determination of the mechanical impact force in the in vitro dissolution test and evaluation of the correlation between in vivo and in vitro release. *International Journal of Pharmaceutics,* 1993, vol. 95, 67-75
- A New Method of Dissolution Testing for Oily Drug Preparations Using an Improved Apparatus. **Y. MACHIDA et al.** Chemical and Pharmaceutical Bulletin. 1986, vol. 34, 2637-2641
- **E. NICOLAIDES et al.** *Pharmaceutical Research,* 1999, vol. 16 (12), 1876-1882
- **J.B. DRESSMAN et al.** *European Journal of Pharmaceutical Sciences,* 2000, vol. 11 (2), 73-80
- **V.H. SUNESEN et al.** *European Journal of Pharmaceutical Sciences,* 2005, vol. 24, 305-313